Europäisches Patentamt

European Patent Office

Office européen des brevets

(11) Publication number: **0 084 448**
**B1**

(12) **EUROPEAN PATENT SPECIFICATION**

(45) Date of publication of patent specification: **04.09.85**

(51) Int. Cl.⁴: **C 07 C 50/04, C 07 C 46/06**

(21) Application number: **83300210.8**

(22) Date of filing: **17.01.83**

(54) **Process for oxidizing phenol to p-benzoquinone.**

(30) Priority: **18.01.82 US 339965**

(43) Date of publication of application:
**27.07.83 Bulletin 83/30**

(45) Publication of the grant of the patent:
**04.09.85 Bulletin 85/36**

(84) Designated Contracting States:
**BE DE FR GB IT NL**

(56) References cited:
**US-A-3 933 681**
**US-A-3 987 068**

(73) Proprietor: **Sun Refining and Marketing Company**
**1801 Market Street**
**Philadelphia, PA 19103 (US)**

(72) Inventor: **Hsu, Chao-Yang**
**615 Farnum Road**
**Media Pennsylvania 19063 (US)**
Inventor: **Lyons, James E.**
**211 Cooper Drive**
**Wallingford Pennsylvania 19086 (US)**

(74) Representative: **Lewin, John Harvey et al**
**Elkington and Fife High Holborn House 52/54**
**High Holborn**
**London WC1V 6SH (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**0 084 448**

**Description**

This invention relates to process for oxidizing phenol to p-benzoquinone.

It is known in the art to oxidize phenol to p-benzoquinone with oxygen in the presence of a copper ion catalyst and such a process is disclosed in US—A—3,987,068. In that disclosure the oxidation is carried out in a nitrile solvent using a complex formed from the copper catalyst and the solvent, and the operating conditions are said to be at temperatures of from about 0° to 100°C and a partial pressure of oxygen of from 7.1 to 202.7, preferably 14.2 to 101.3 bar (about 7 to 200, preferably 14 to 100, atmospheres). As pointed out in US—A 3,987,068, the yield of quinone product increases with increased partial pressure of oxygen and it appears from the data therein to require partial pressures of oxygen above 101.3 bar (about 100 atmospheres) in order to achieve conversions of phenol to p-benzoquinone of the order of about 75%. Such pressures are too high to be useful in an economical commercial process because they require special equipment of high capital cost.

US—A—3,870,731 relates to the oxidation of phenols to benzoquinones in the presence of copper salts as catalysts where the catalyst is promoted with thiocyanate, cyanate, cyanide and halogen ions. In such reactions, a solvent such as water is disclosed and other polar solvents soluble or miscible with water may be used. Such solvents are exemplified as certain amides, alcohols, and sulphoxides. It is also gratuitously stated that any of the various solvents may be used alone or in combination with water in any desired ratio. There is no teaching, however, that water may be used to modify the cuprous ion (but not cupric ion) catalyst to obtain increased selectivity in accordance with this invention. Furthermore, in our experience we find that water alone is not a suitable solvent for the reaction.

It has now been found that the copper-catalyzed process for oxidation of phenol to p-benzoquinone can be significantly improved so as to enable operation at lower, commercially useful pressures and still achieve an improved selectivity to product. In accordance with the invention, such objectives are achieved by conducting the oxidation of phenol in the presence of a monovalent copper ion catalyst (e.g. Cu$^+$) which is modified with water.

The effect of enhancing the yield of p-benzoquinone with water in the presence of a cuprous ion catalyst is particularly surprising because water has no such effect with a cupric ion catalyst, although water is effective with a cupric ion catalyst when an alkali metal base is also present as disclosed in our related U.S. application S.N. 284,893 filed August 20, 1981 (European Application No. 82 303639.7, EP—A 0070665).

In carrying out the process of the invention, moderate temperature conditions, conventional solvent systems and a choice of monovalent copper catalyst may be used. Thus, a temperature of from about 60° to about 85°C (preferably about 65°C) and a solvent, preferably a nitrile such as acetonitrile, are usually employed. The copper catalyst will be preferably a copper (I) halide, preferably chloride, although nitrate is operable and mixtures of such salts also may be used. However, other copper (I) salts such as oxide, thiophenoxide, acetate and carboxylates, have been found not to be effective catalysts for the reaction. As indicated above, the reaction can be carried out at moderate pressures and such pressures will generally be 6.9 to 34 bar gauge (about 100 to about 500 psig) partial pressure of oxygen, preferably 13.8 to 27.6 bar gauge (about 200 to 400 psig). Mixtures of oxygen and nitrogen, air alone, or oxygen alone may be used, but preferably mixtures of oxygen and nitrogen such as air will be employed as the oxygenating medium.

The addition of any amount of water to the reaction mass over the normal temperature range of operation will be beneficial, but it will be understood that optimum benefit will be obtained under certain specific parameters. Thus, although the ratio of added water to phenol used will vary preferably from about 1.0 to about 4.0 moles of water per mole of phenol over the operating temperature range, optimum results are obtained at about 65°C and at a water to phenol ratio of from about 1.25 to about 2.50 moles of water per mole of phenol.

In a most preferred embodiment of the invention, a mixed solvent system of water (about 16% by weight) and acetonitrile (about 84%) will be used. Such a solvent system not only enables selectivity of over 90% to be obtained, but also is advantageous in that it is a low boiling azeotrope (76.5°C) which makes possible solvent recycle with lower energy input.

In order to further illustrate the invention, the following Examples are given:

**Example 1**

A solution of phenol in 5 ml of acetonitrile at 65°C which was agitated in a magnetically stirred mini-autoclave under an initial total pressure of 52 bar gauge (750 psig) was oxidized over a three hour period with a mixture of 40% (vol.) oxygen and 60% nitrogen in the presence of 0.55 mmole of copper catalyst. The reaction parameters and results obtained by standard GLPC are shown in the following Table I.

2

# 0 084 448

### TABLE I
### Effect of water on copper-catalyzed oxidation of phenol
#### Anal. of reaction mixture (mmoles)

| Catalyst | Phenol (mmoles) | $H_2O$ (mmoles) | PhOH[a] | PBQ[b] | OCP[c] | PCP[d] | Conv. (%) | Sel. (%) |
|---|---|---|---|---|---|---|---|---|
| $CuCl_2$ | 8 | 0 | | | | | 73 | 49 |
| $CuCl_2$ | 8 | 14 | | | | | 35 | 48 |
| CuCl | 16 | 0 | 8.48 | 4.57 | — | 0.10 | 47 | 61 |
| CuCl | 16 | 14 | 10.21 | 4.77 | 0.01 | 0.07 | 36 | 82 |

[a]=Phenol
[b]=p-Benzoquinone
[c]=o-Chlorophenol
[d]=p-Chlorophenol.

As can be seen from the data in Table I, only with the cuprous ion catalyst does the presence of water show improved selectivity.

### Example II

Phenol was dissolved in 5 ml of acetonitrile and 0.55 mmole of the catalyst and water was added. The mixture was stirred and then pressured to 52 bar (750 psi) with a mixture of 40% $O_2$/60% $N_2$. The mixture was heated under pressure for 3 hours and the products analyzed. Table II indicates the reaction conditions and results obtained.

3

TABLE II

| PhOH (mmoles) | H$_2$O (ml) | H$_2$O (moles per mole phOH) | Temp. (°C) | Conversion of phenol (mole %) | Selectivity to p-benzo-quinone (%) |
|---|---|---|---|---|---|
| 8 | None | — | 65 | 90 | 54 |
| | 0.125 | 0.88 | | 78 | 71 |
| | 0.250 | 1.75 | | 61 | 78 |
| | 0.375 | 2.63 | | 51 | 85 |
| | 0.500 | 3.50 | | 46 | 81 |
| 16 | None | — | 65 | 41 | 64 |
| | 0.125 | 0.44 | | 52 | 80 |
| | 0.250 | 0.88 | | 37 | 83 |
| | 0.375 | 1.32 | | 34 | 89 |
| | 0.500 | 1.75 | | 28 | 98 |
| 8 | 0.500 | 3.50 | 65 | 46 | 81 |
| 12 | | 2.33 | | 32 | 95 |
| 16 | | 1.75 | | 28 | >95 |
| 20 | | 1.40 | | 21 | >95 |
| 8 | 0.500 | 3.50 | 75 | 60 | 69 |
| 12 | | 2.33 | | 47 | 84 |
| 16 | | 1.75 | | 38 | 85 |
| 20 | | 1.40 | | 31 | 80 |
| 8 | 0.500 | 3.50 | 85 | 71 | 76 |
| 12 | | 2.33 | | 60 | 72 |
| 16 | | 1.75 | | 46 | 76 |
| 20 | | 1.40 | | 36 | 79 |
| 8 | 0.500 | 3.50 | 95 | 83 | 65 |
| 12 | | 2.33 | | 66 | 71 |
| 16 | | 1.75 | | 57 | 68 |
| 20 | | 1.40 | | 48 | 49 |
| 8 | 1.00 | 7.00 | 95 | 68 | 63 |
| 12 | | 4.66 | | 55 | 67 |
| 16 | | 3.50 | | 45 | 72 |
| 20 | | 2.80 | | 35 | 76 |

Example III

Phenol (16 mmole) was dissolved in 5 ml water and 0.55 mmole of CuCl was added. The mixture was stirred and then pressured to 52 bar (750 psi) with a mixture of 40% O$_2$/160% N$_2$. The mixture was heated under pressure at 65°C for 3 hours and the products analyzed. Conversion of phenol was 28.0% and selectivity to p-benzoquinone was only 8.2% giving a total yield of 2.3% p-benzoquinone. Thus, water alone is not a suitable solvent for the reaction when cuprous ion is used.

Example IV (Using CH$_3$CN—H$_2$O azeotrope)

The following were charged into a 500 ml stainless steel autoclave phenol, 60.23 g (640 mmole), cuprous chloride, 2.2 g (22 mmole), acetophenone 8.0 ml (68 mmol) and 220 ml of a mixture of acetonitrile and water having the composition: 84% CH$_3$CN and 16% H$_2$O by weight which corresponds to the azeotrope boiling at 76°C. The oxidizing gas, a mixture of 40% O$_2$ and 60% N$_2$, was sparged through the reactor at 25 ml/min while maintaining a pressure of 52 bar gauge (750 psig) on the reactor contents. The reactor was heated to 65°C and the mixture stirred at this temperature with constant sparging for 5 hours. After this time standardized gas chromatographic analysis showed that 318.3 mmoles of phenol remained unreacted and 299.9 mmoles of p-benzoquinone had been produced. This corresponds to a phenol conversion of 50.3% and a selectivity to p-benzoquinone of 93.2%.

Solvent recovery is effected in the above system by distilling off the low boiling azeotrope and the distillate is recycled for use as solvent in subsequent reactions.

**Claims**

1. A process for oxidizing phenol to benzoquinone in the presence of a monovalent copper salt catalyst selected from halides and nitrate, characterised in that increased selectivity is obtained by modifying the catalyst with water.

2. A process as claimed in claim 1, which is carried out in a nitrile solvent.

3. A process as claimed in claim 1 or 2, wherein the catalyst is cuprous chloride.

4. A process as claimed in any of claims 1 to 3, wherein the process is carried out at a temperature of from 60° to 85°C.

5. A process as claimed in any of claims 1 to 4, wherein the water is present in a ratio of from 1.0 to 4.0 mmoles per mmole of phenol.

6. A process as claimed in any of claims 1 to 5, which is carried out in an acetonitrile solvent system.

7. A process as claimed in any of claims 1 to 6, wherein the process is carried out at about 65°C and the water is present in a ratio of from 1.25 to 2.50 moles per mole of phenol.

8. A process as claimed in any of claims 1 to 7, carried out in a solvent system which is a mixture of 84% by weight of acetonitrile and 16% water.

9. A process as claimed in claim 8, wherein the solvent is azeotropically distilled off for recycle.

## Patentansprüche

1. Verfahren zur Oxidation von Phenol zu Benzochinon in Gegenwart eines einwertigen Kupfersalzes, das ausgewählt ist aus Halogeniden und Nitraten, als Katalysator, dadurch gekennzeichnet, daß durch Modifizieren des Katalysators mit Wasser erhöhte Selektivität erzielt wird.

2. Verfahren nach Anspruch 1, welches in einem Nitril-Lösungsmittel durchgeführt wird.

3. Verfahren nach Anspruch 1 oder 2, bei dem der Katalysator Kupfer-I-chlorid ist.

4. Verfahren nach einem der Ansprüche 1 bis 3, welches bei einer Temperatur von 60 bis 85°C durchgeführt wird.

5. Verfahren nach einem der Ansprüche 1 bis 4, bei dem das Wasser in einem Anteil von 1,0 bis 4,0 mMol pro mMol des Phenols vorhanden ist.

6. Verfahren nach einem der Ansprüche 1 bis 5, welches in einem Acetonitril-Lösungsmittelsystem durchgeführt wird.

7. Verfahren nach einem der Ansprüche 1 bis 6, welches bei etwa 65°C durchgeführt wird und bei dem das Wasser in einem Anteil von 1,25 bis 2,50 Mol pro Mol des Phenols vorhanden ist.

8. Verfahren nach einem der Ansprüche 1 bis 7, welches in einem Lösungsmittelsystem durchgeführt wird, das ein Gemisch aus 84 Gew.-% Acetonitril und 16% Wasser ist.

9. Verfahren nach Anspruch 8, bei dem das Lösungsmittel azeotrop abdestilliert wird, um es zurückzuführen.

## Revendications

1. Procédé d'oxydation de phénol en benzoquinone, en présence d'un catalyseur de sel de cuivre monovalent, choisi à partir des halogénures et nitrate, caractérisé en ce qu'on obtient une sélectivité accrue par modification du catalyseur avec de l'eau.

2. Procédé selon la revendication 1, que l'on effectue dans un solvant nitrile.

3. Procédé selon la revendication 1 ou 2, dans lequel le catalyseur est du chlorure cuivreux.

4. Procédé selon une des revendications 1 à 3, au cours duquel on opère à une température comprise entre 60° et 85°C.

5. Procédé selon une des revendications 1 à 4, dans lequel l'eau est présente à raison de 1 0 4 mMoles par mMole de phénol.

6. Procédé selon une des revendications 1 à 5, dans lequel on opère dans un système solvant à l'acétonitrile.

7. Procédé selon une des revendications 1 à 6, au cours duquel on opère à 65°C environ, en présence de 1,25 à 2,50 moles d'eau par mole de phénol.

8. Procédé selon une des revendications 1 à 7, au cours duquel on opère dans un système solvant qui est un mélange pondéral de 84% d'acétonitrile et de 16% d'eau.

9. Procédé selon la revendication 8, au cours duquel le solvant est chassé par distillation azéotropique en vue de son recyclage.